# EUROPÄISCHE PATENTSCHRIFT

(11) **EP 0 913 129 B1**
(45) Veröffentlichungstag und Bekanntmachung des Hinweises auf die Patenterteilung: **27.08.2003**
(21) Anmeldenummer: 97118789.3
(22) Anmeldetag: 29.10.1997
(51) Int. Cl.: A61B 17/72

(54) **Osteosynthetischer Wendeldraht**
Osteosynthetic helical wire
Fil hélicoidal ostéosynthétique

(43) Veröffentlichungstag der Anmeldung: 06.05.1999
(73) Patentinhaber: Laminger, Karl, Dr.med., 2345 Brunn/Gebirge (AT)
(72) Erfinder: Hasenbacher, Walter, Dr. med., 1130 Wien (AT); Laminger, Karl, Dr. med., 1230 Wien (AT)
(74) Vertreter: Haffner, Thomas M., Dr.

(56) Entgegenhaltungen:
- WO-A-97/07744
- DE-A- 3 835 682
- DE-U- 9 412 040
- US-A- 5 626 613

## Beschreibung

Die Erfindung betrifft ein Osteosynthesehilfsmittel zur Versorgung kopfnaher Oberarmfrakturen in Form einer in den Markraum einführbaren, elastisch verformbaren Wendel aus z.B. Implantatstahldraht, wobei die Wendel als selbstschneidende Schraubenfeder ausgeführt ist. Einrichtungen dieser Art sind bereits aus der EP 374 088 A1 und DE 3835 682 A1 bekanntgeworden.

Das Deutsche Gebrauchsmuster 94 12 040 (Basis für den Oberbegriff der Anspruchs 1) offenbart eine Humerusspirale zum Fixieren der abgebrochenen Schulterkugel am Oberarmknochen, bestehend aus einem geraden Draht, dessen vorderes ca. 2 bis 6 cm langes Teil korkenzieherartig gewunden ist. Die WO 97/07744 zeigt und beschreibt einen helixförmigen Anker zur Fixierung von organischem Gewebe, der auch dazu verwendet werden kann, als Schraubenersatz Knochenteile miteinander zu verbinden.

In bestimmten Fällen ist die Knochensubstanz, in welche ein Bohrdraht einzubringen ist, so weich, daß es schwierig ist, ihn ausreichend zu fixieren.

Aufgabe der Erfindung ist es, ein Osteosynthesehilfsmittel zur Versorgung kopfnaher Oberarmfrakturen zu schaffen, das einerseits einen geringen operativen Aufwand erfordert, andererseits eine ausreichende Verankerung in dem - vornehmlich in vorgerücktem Lebensalter - weichen Knochen des Oberarmkopfes gewährleistet.

Diese Aufgabe wird durch einen Osteosyntheschilfsmittel gemäß Anspruch 1 gelöst.

Wendelig ausgeführte Schraubenfedern zur Versorgung von Frakturen im Schaftbereich langer Röhrenknochen sind - wie schon oben erwähnt - bekannt. Sie stabilisieren durch den langstreckigen Kontakt mit der Wand der Markhöhle, besitzen eine hohe Biegesteifigkeit sowie Längsrigidität und werden im Sinne von Marknägeln in axialer Richtung eingebracht. Dies ist oft nur mit Eröffnung der angrenzenden Gelenke möglich, was einen erheblichen operativen Aufwand darstellt und besonders im osteoporotischen Knochen das Risiko einer Zerstörung des artikulären Segmentes in sich birgt. Ein deutlich geringerer Aufwand liegt vor, wenn das Implantat durch ein im Schaft liegendes gelenksund frakturfernes Zugangsloch eingebracht werden kann. Dies wird erfindungsgemäß dadurch gelöst, daß die Wendel mit speziellen Materialeigenschaften ausgestattet wird, die nach dem Einbringen eine gleichmäßige, reversible Steigungsänderung des Gewindes erlaubt, um eine bis zu 90°ige Richtungsänderung zu gestatten. Die aus Konfiguration und Materialeigenschaften resultierende Rigidität der Wendel muß andererseits so groß sein, daß ein gleichmäßiger Rotationskraftfluß ohne bleibende Verformung möglich ist. Nachdem die Spitze der Wendel nach Richtungsänderung die ursprüngliche Form wieder eingenommen hat, wird das Implantat im Sinne einer an sich bekannten korkenzieherartig ausgebildeten kernlosen Knochenschraube in den spongiösen Knochen des Oberarmkopfes eingebohrt.

Die am Eintrittsbohrloch bleibende, an sich reversible Verformung, gewährleistet den gewünschten Schutz vor Implantatwanderung.

Im Folgenden wird die Erfindung anhand der Zeichnung näher erläutert.
Figur 1 ist eine Ansicht des Wendeldrahtes.
Figur 2 zeigt eine Ansicht in axialer Richtung.
Figur 3, 4 und 5 geben verschiedene Querschnitte für den Wendeldraht wieder.
Figur 6 gibt die Verformung des Drahtes im Oberarmknochen in Folge der Dreipunktverspannung wieder.

Die Wendel (1) wird durch ein distal der Fraktur gelegtes kleines Bohrloch am lateralen Oberarmschaft (FIG. 6, C) in proximaler Richtung so eingedreht, daß sie unter elastischer Verformung in proximaler Richtung fortbewegt wird, die Fraktur überbrückt und in weiterer Folge selbst schneidend-wendelig in den spongiösen Knochen des Oberarmkopfes (FIG. 6, A) einbohrt.
Durch dieses Vorgehen wird die Wendel (1) im Sinne einer Feder an drei Punkten abgestützt: 1. in der Spongiosa des Oberarmkopfes (FIG. 6, A), 2. am Eintrittsbohrloch (FIG. 6, C), 3. an der dem Eintrittsbohrloch gegenüberliegenden Wand des Oberarmschaftes (FIG. 6, B).

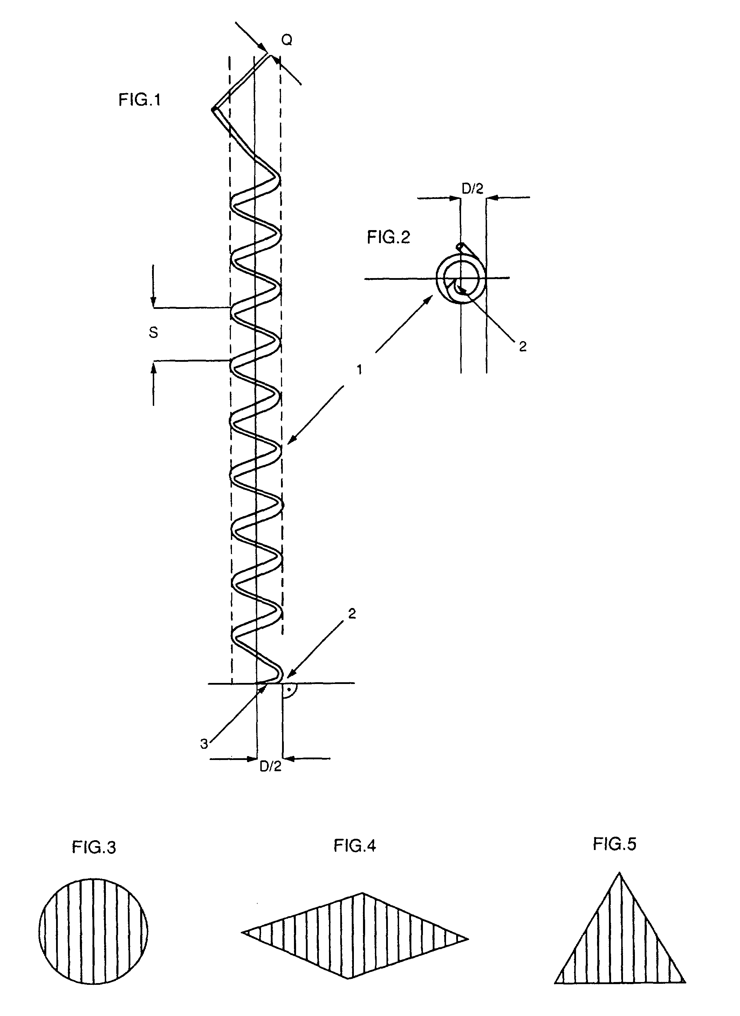

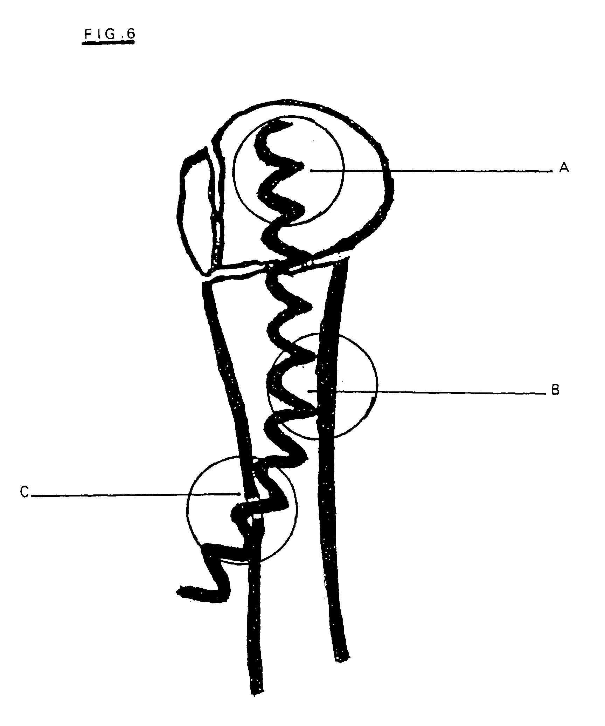

Die oben beschriebenen Eigenschaften werden vorzugsweise durch die Verwendung von rostfreien Edelstählen wie z.B. X 12 CrNI 17/7 bei einem Drahtquerschnitt von 1,4 bis 2,2 mm - bei einem Wendelaußendurchmesser von 8 bis 14 mm sowie durch ein Design wie folgt erreicht:
□ Querschnitt (Q) des Drahtes rund (FIG. 3), rhombisch (FIG. 4) oder dreieckig (FIG. 5)
   Der runde Querschnitt des Drahtes vermeidet Spitzenbelastungen im Knochen, bietet aber weniger günstige Führungseigenschaften als die anderen genannten Querschnitte.
□ Die Steigung der Wendelung (S) ist gleich oder größer (bis zu einem Verhältnis 1: 2,8) als der halbe Außendurchmesser (D/2) der Wendel (1) (FIG. 1, 2)
□ Um den Effekt der Dreipunktverspannung (FIG. 6 A, B, C) zu ermöglichen, ist eine Mindestlänge der Wendel (1) von 7,5 cm erforderlich.
□ Die Spitze (2) des Wendeldrahtes muß, unabhängig vom benützten Querschnitt, einen Schliff (3) in Richtung "Kern" der Wendel besitzen, um einerseits ein Verhaken an der Oberfläche der Markraumhöhle zu vermeiden und andererseits ein Einbohren in die Spongiosa des Oberarmkopfes (FIG. 6, A) möglich zu machen.

## Patentansprüche

1. Osteosynthesehilfsmittel zur Versorgung kopfnaher Oberarmfrakturen mit einer in den Markraum einführbaren, elastisch verformbaren Wendel aus z.B. Implantatstahl-Draht, wobei die Wendel als selbstschneidende Schraubenfeder ausgeführt ist, **dadurch gekennzeichnet, dass** die Wendel (1), zu deren von lateral erfolgenden Einführung in den Markraum über ihre ganze Länge bis zu 90° in Längsrichtung reversibel verformbar ist, wobei diese Verformbarkeit innerhalb einer Strecke, die dem doppelten Durchmesser der Wendel (1) entspricht, ermöglicht ist, dass die Länge der Wendel mindestens 7,5 cm, der Querschnitt des Drahtes 1,4 mm bis 2,2 mm, der Wendelaußendurchmesser 8 mm bis 14 mm und das Verhältnis des halben Wendelaußendurchmessers zur Steigung der Wende 1:1 bis 1:2,8 beträgt.

2. Osteosynthesehilfsmittel nach Patentanspruch 1 **dadurch gekennzeichnet, dass** die Spitze (2) der Wendel (1) unabhängig vom gewählten Draht-Querschnitt einen senkrecht zur Längsachse in Richtung Wendelkern verlaufenden Schliff (3) aufweist.

## Claims

1. An osteosynthesis aid for treating upper arm fractures close to the head, comprising an elastically deformable helix capable of being inserted into the medullary space and made, for instance, of implant steel wire, said helix being configured as a self-cutting coil spring, **characterized in that** the helix (1) for its lateral introduction into the medullary space is reversibly deformable in the longitudinal direction by up to 90° over its entire length, said deformability being feasible within a distance corresponding to twice the diameter of the helix (1), the helix having a minimum length of 7,5 cm, a wire cross section of 1,4 to 2,2 mm, a helix external diameter of 8 to 14 mm and a ratio of 1:1 to 1:2,8 of half the external diameter of the helix to the pitch of the helix.

2. An osteosynthesis aid according to claim 1, **characterized in that** the tip (2) of the helix (1) irrespective of the selected wire cross section has a ground surface (3) extending perpendicular to the longitudinal axis in the direction of the core of the helix.

## Revendications

1. Moyen auxiliaire d'ostéosynthèse pour le traitement de fractures de l'humérus proches de la tête, avec un serpentin déformable élastiquement constitué par exemple d'un fil d'acier pour implant, pouvant être introduit dans le canal médullaire, le serpentin étant réalisée sous la forme d'un ressort hélicodïal autocoupant, **caractérisé en ce que** pour son introduction dans le canal médullaire, s'effectuant depuis le côté, le serpentin est déformable de manière réversible dans la direction longitudinale sur toute la longueur, jusqu'à 90°, cette déformabilité étant rendue possible à l'intérieur d'une distance qui correspond au double du diamètre du serpentin (1) et que la longueur du serpentin est au moins de 7,5cm, la section du fil métallique de 1,4m à 2,2mm, le diamètre extérieur du serpentin de 8mm à 14mm et le rapport entre la moitié du diamètre extérieur du serpentin et le pas d'enroulement est de 1:1 à 1:2,8.

2. Moyen auxiliaire d'ostéosynthèse selon la revendication 1, **caractérisé en ce que** la pointe (2) du serpentin (1) présente, indépendamment de la section choisie pour le fil métallique, un biseau (3) s'étendant perpendiculairement à l'axe longitudinal dans la direction du noyau du serpentin.
